# EUROPEAN PATENT SPECIFICATION

(11) **EP 0 353 315 B1**
(45) Date of publication and mention of the grant of the patent: **13.10.1993**
(21) Application number: 88112448.1
(22) Date of filing: 01.08.1988
(51) Int. Cl.: A61B 5/02

(54) **Method for measuring blood pressure and apparatus for automated blood pressure measuring**
Verfahren und Vorrichtung zur automatischen Blutdruckmessung
Procédé et appareil pour la mesure automatique de la pression sanguine

(43) Date of publication of application: 07.02.1990
(73) Proprietor: Hewlett-Packard GmbH, D-71004 Böblingen (DE)
(72) Inventor: Frankenreiter, Michael, D-7032 Sindelfingen (DE)
(74) Representative: Kurz, Peter

(56) References cited:
- EP-A- 0 079 305
- EP-A- 0 208 520
- DE-A- 3 424 535
- US-A- 4 349 034

## Description

The present invention relates to a method for measuring blood pressure, comprising the steps of applying a blood presure cuff about a subject's limb containing an artery; inflating said cuff to a pressure pₘₐₓ above the systolic pressure p_{sys}, thereby occluding said artery, reducing cuff pressure from pₘₐₓ to at least a pressure pₘᵢₙ below the diastolic pressure p_{dia}, thereby permitting an increasing flow through the progressively less occluded artery, conducting said reducing of cuff pressure with definite steps Δ p_{def}, detecting of effects at the cuff caused by said increasing flow through said progressively less occluded artery, processing said detected effects in processing means and displaying said processed effects as subject's actual blood pressure values, and conducting a next subsequent blood pressure measuring cycle cᵢ₊₁ after a time interval.

The invention further relates to an apparatus for automated blood pressure measuring, in particular for cyclic measuring, comprising an inflatable and deflatable pressure cuff, said cuff being applicable about a subject's limb containing an artery, means for inflating said cuff to a pressure pₘₐₓ above the systolic pressure p_{sys}, thereby occluding said artery, means for reducing cuff pressure from pₘₐₓ to at least a pressure pₘᵢₙ below the diastolic pressure p_{dia}, thereby permitting an increasing flow through the progressively less occluded artery, said means for reducing allow a stepwise reduction of a cuff pressure, thereby stepwise deflating said cuff, means for detecting of effects at the cuff, caused by said increasing flow through said progressively less occluded artery, processing means for processing said detected effects and for converting said effects to subject's actual blood pressure values, and means for initiating a subsequent next measuring cyle cᵢ₊₁.

Such method and such an apparatus are known from EP-A2-0 208 520.

During such so-called "non-invasive" blood pressure measurements, an inflatable cuff is suitably located around a limb of a subject, for example a human, and is inflated up to a predetermined pressure pₘₐₓ above a systolic pressure p_{sys}, thereby occluding an artery. The limb may be an arm, in particular the upper arm, a foot, or a finger of a subject. Thereupon, cuff pressure is reduced with definite steps Δ p_{def}. Such definite steps Δ p_{def} are, for example, on measuring a human adult 1,064 kPa(8 mmHg) or 0,532 kPa(4 mmHg) for neonates. During deflating of cuff, an increasing flow through the progressively less occluded artery is permitted. The effect at the cuff may be detected by the so-called "oscillometric method", for example as described within EP-A2-0 208 520.

The oscillometric method of measuring blood pressure is one of the most popular methods in commercially available systems. This method relies on measuring changes in arterial counterpressure, such as imposed by the inflatable cuff. At each pressure level after each cuff pressure reducing step, fluctuations are monitored. The resulting signals typically consist of the DC voltage with small superimposed variational components caused by the arterial blood pressure pulsations. After suitable filtering to reject the DC component and to provide amplification, peak pulse amplitudes above a given base line are measured are stored. As deflating of cuff continues, the peak amplitudes will normally increase from a lower level to a relative maximum, and thereafter will decrease. The lowest cuff pressure at which the oscillation has a maximum value is representative of mean arterial pressure. Systolic and diastolic pressures p_{sys}, p_{dia} can be derived as a predetermined fraction of mean arterial pressure, or by more sophisticated methods of direct processing of the oscillatory complexes.

In some applications, several subsequent measurement cycles are necessary, for example in the so-called "automatic mode" or in the "stat mode", and become essential aspect of human and veterinary treatment. Such subsequent measurements are preferably conducted in emergency rooms, intensive and critical care units, and in the operation theatre.

One problem on conducting said reducing of cuff pressure with definite steps Δ p_{def}, for example with 1,064 kPa(8 mmHg) steps is that the number of steps depends on the difference between the systolic pressure p_{sys} and the diastolic pressure p_{dia} of the human individual.

A human adult may be characterized by his blood pressure values expressed in a pair of numbers representative to the systolic and diastolic pressures.

For example,
120/80, adult A, the so-called average human,
70/50, adult B, with hypotension,
110/40, adult C, for example a sportsman,
180/100, adult D, with hypertension.

The measuring of adult A needs seven steps, adult B only four steps, adult C eleven steps, and adult D twelve steps. The result of the adult B measurement based on only four steps is inaccurate and may deviate considerably from the actual values. The measurements of adults C and D are unnecessary long. The adult A measurements with about seven steps are of short time and are most convenient for a human, and are sufficient for obtaining a good result with minimum deviation of the actual values. However, adults of kind A represent the minor parts of humans, at which blood measurements are usually conducted.

The method as described within EP-A2-0 208 520 uses non-uniform pressure decrementing steps for reducing the overall time for conducting a measurement cycle. The apparatus described within EP-A2-0 208 520 uses valves for deflating cuff having differently sized orifices. As long as the cuff pressure is relatively high, the deflation velocity through a smaller orifice valve is high. If cuff pressure has reduced to a particular pressure value, a second orifice is opened, thereby allowing a quicker deflation. As a result, a shorter overall measuring time is possible.

However, this method and apparatus depends on the structure of the cuff to be used and not to the subject to be measured. Therefore, the actual condition of subject to be measured, in particular, changes of condition during a measurement cycle in the so-called stat mode, are not taken into consideration. As a result, different numbers of deflating steps are conducted, depending on the systolic and diastolic pressure of subject. As a result, measurements with only a few deflating steps are of less relevance than measurements with more deflating steps. However, numerous deflating steps may be unnecessary, and such great number of steps being disagreeable for subject to be measured.

The US-A-4 349 043 discloses an automatic indirect blood pressure reading method using an apparatus that automatically and adaptively pumps up an arm cuff to a proper pressure by taking the previous cuff pressure measurement and adding approximately 60 mm of mercury to the old pressure before beginning measurement of the amplitude of the oscillations in the cuff. This adaptive pump-up feature minimizes the period of time that blood flow is occluded in the arm by minimizing the amount of overpressure required to occlude. The size of the decremental steps Δ p_{def} of a certain measuring cycle is of constant value, i.e. all decremental cuff deflating steps are of identical size. If the systolic pressure is relatively high and the diastolic pressure is relatively low, a great number of decremental steps has to occur during a measuring cycle. This method has the disadvantage that in case the referenced pressure value of a previous measuring cycle, for example the maximum arterial pressure MAP, is relatively high, an adding of approximately 60 mm of mercury to the old pressure leads to a great number of incremental steps of identical size in a later measuring cycle, if the patient MAP value is lower than in the previous measurement cycle. Such great number of unnecessary deflating steps being disagreeable for the patient to be measured.

The EP-A-0 079 305 discloses a method for measuring blood pressure by conducting a first measuring cycle for determining the approximate values of the systolic and the diastolic pressure followed by a second measuring cycle with several small incremental steps Δ p_{def} in proximity of the systolic pressure and in the proximity of the diastolic pressure. This method leads to an exact determination of systolic and diastolic pressure values, however, this method is not useful for periodical measurements because of the great number of incremental steps which lead to a relative long-time measurement cycle being disagreeable for the patient.

A similar method is known from DE-A-3 424 535. The deflating speed of the cuff is very slow in the range of the systolic and the diastolic pressure. In the range between the systolic and the diastolic pressure, the deflating speed is relatively high. Changes of the deflating speeds are very disagreeable for the patient.

It is, therefore, object of the present invention to provide a method and an apparatus for measuring blood pressure as initially indicated allowing convenient measurements to subjects having different individual bood pressure values.

This object is achieved by a method in which the size of the decremental steps Δ p_{def} of a certain measuring cycle cᵢ₊₁ is a function of subject's actual blood pressure of a preceding measuring cycle cᵢ and which step Δp_{def} is constant within said measuring cycle cᵢ₊₁. This object is further achieved by an apparatus having processing means, further comprising storing means for storing pressure values of subject within a cycle cᵢ, said processing means provide for said means for reducing cuff pressure the size of decremental steps Δ p_{def} of cycle cᵢ₊₁ as a function of said stored pressure values of cycle cᵢ.

According to the invention, the size of the decremental steps Δ p_{def} of cycle cᵢ₊₁ is adapted to subject's actual blood pressure values of the preceding cycle cᵢ and which step Δp_{def} is kept constant within said measuring cycle cᵢ₊₁.

The pressure values of the preceding cycle cᵢ indicate what kind of subject is to be measured, i.e. a subject with hypertension, normal blood pressure or hypotension. The size of deflating steps Δ p_{def} can now be adapted in such a manner that at the one side a convenient number of deflating steps and at the other side a sufficient number of steps for obtaining measurement results with high relevance are available. Furthermore, if actual blood pressure values will shift during a measurement cycle cᵢ in the stat mode, for example based on a drug being applied to the subject, the deflating steps in cycle cᵢ₊₁ are adapted to such shifts, too. As a result, blood pressure measurings of high relevance are achieved, said measurings being agreeable to all different kinds of subjects to be measured.

According to another aspect of the invention, the size of decremental steps in cycle cᵢ₊₁ is 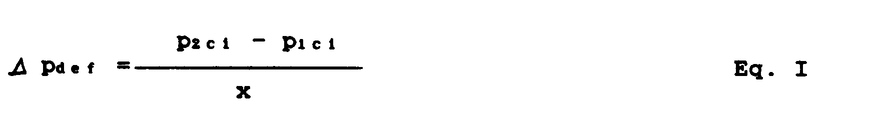
with
p_{2ci} ≦ pₘₐₓ in cycle cᵢ
p_{1ci} ≧ pₘᵢₙ in cycle cᵢ,
p_{2ci} > p_{1ci} and x = constant

This has the advantage that the size of steps Δ p_{def} depends on two pressure values, being typical for the individual subject to be measured and being typical for the condition of the subject in the preceding measurement cycle cᵢ. The constant factor x leads to the same number of deflating or incremental steps Δ p_{def} during deflating cuff from pressure p_{2ci} to pressure p_{1ci}. Therefore, each particular blood pressure value, for example the systolic pressure p_{sys}, of each cycle within a lot of cycles in the stat mode is of the same relevancy. Additionally, both, measuring a subject with hypertension or hypotension, occur with the same number of incremental steps, at least within the interval p_{2ci} - p_{1ci}. As a result, measurements at different kinds of blood pressure type subjects give the same relevancy of values.

According to another aspect of the invention,
p_{2ci} = p_{sysci},
p_{1ci} = p_{diaci} and
3 ≦ x ≦ 6.

This has the advantage that two particular pressure values are evaluated for determining the size of the incremental steps, said particular values p_{sysci} and p_{diaci} are essential ones for indicating the condition of subject in cycle cᵢ. Conducting three up to six deflating steps between the systolic and diastolic pressure leads to agreeable measuring times per cycle on using conventional oscillometric methods, in particular if measuring a human, and result in pressure values of high relevancy in view of the actual values.

According to another aspect of the invention, the size of decremental steps Δ p_{def} in a first cycle c₀ is adapted to pre-known subject's blood pressure data being the basis of decremental steps in cycle c₀.

This has the advantage that, in particular if using the automatic mode, the first cycle c₀ is adapted to known blood pressure values obtained on earlier measurements. However, if no pressure values of subject are known, within the first cycle c₀ a predetermined size of incremental steps Δ p can be used.

According to another aspect of the invention, Δ p_{def} can vary within
Δ p_{defmin} ≦ Δ p_{def} ≦ Δ p_{defmax}.

This has the advantage that in cases of measuring subjects having extremely individual pressure values, the size of decremental steps becomes neither too great nor too small.

According to another aspect of the invention, in particular if measuring a human,
Δ p_{defmin} is about 0,5 kPa(4 mmHg), and
Δ p_{defmax} is about 2 kPa(16 mmHg).

This has the advantage that the size of incremental pressure steps varies within limits being agreeable to any human, i.e. an adult or a neonate.

Some embodiments of the present invention will now be described with reference to the accompanying drawings in which
- Figure 1: shows a graph having plotted a cuff pressure via time, demonstrating a first embodiment according to the invention,
- Figure 2: shows a similar graph in Figure 1, demonstrating a second embodiment according to the invention.

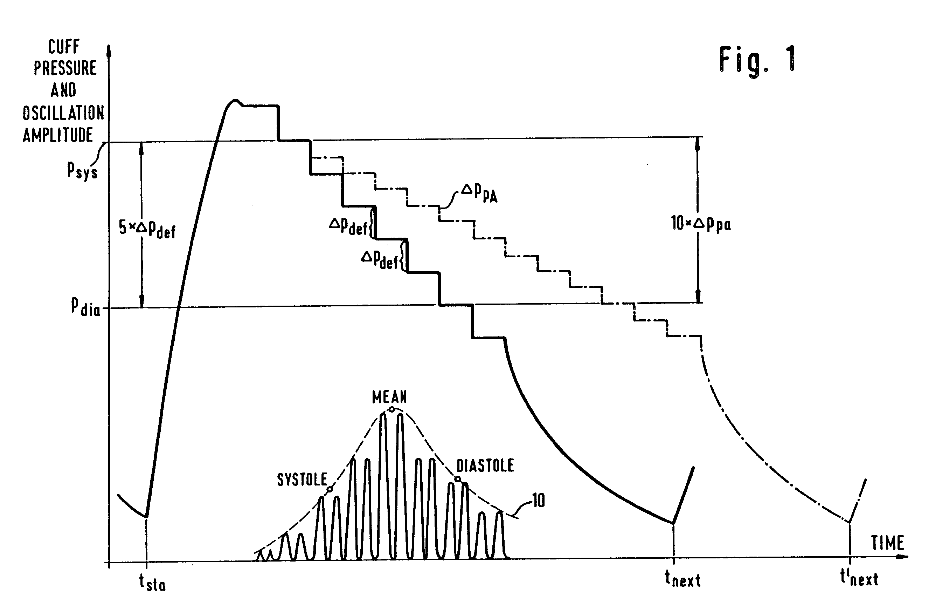

Referring now to Figure 1, the graph shown is representative to a cuff pressure/time graph of a non-invasive blood pressure measurement. A blood pressure cuff is applied about a subject's artery and inflated above the systolic level p_{sys}, thus fully occluding the artery for a full heart cycle. The cuff pressure is thereafter reduced step by step to permit an increasing flow through the progressively less occluded artery (upper graph in Figure 1 starting at time tₛₜₐ).

In accordance with conventional oscillometric techniques, pressure oscillation in the artery are sensed by changes in the counterpressure of the cuff, and, in turn, by a transducer. A measure of the peak amplitudes of the successively encountered oscillary complexes (not shown in the upper graph) are stored in a memory. Also retained is the cuff pressure obtained for each complex peak. As the measurement cycle processes, the peak amplitude of the blood pressure complexes generally become monitonically larger to a maximum and then become monitonically smaller as the cuff pressure continues toward deflation. The peak amplitude of the cuff pressure oscillation complexes and the corresponding occluding cuff pressure values are retained in a computer memory of the processing means.

The inflating of cuff occurs within two or four seconds, depending on cuff size and arrangement of cuff, e.g. about subject's upper arm or finger.

For interpretation of the peak amplitudes (within each step two amplitudes are measured), an envelope 10, surrounding the peak amplitudes, is determined (shown within Figure 1 in the lower graph).

Within Figure 1, the dash-dotted line demonstrates a conventional oscillometric method using predetermined sizes of incremental deflating steps Δ pₚₐ. The size of steps Δ pₚₐ is 1,064 kPa(8 mmHg). The dash-dotted line is representative to an adult D with hypertension typical blood pressure values of 180/100. As a result, ten deflating steps Δ pₚₐ between p_{sys} and p_{dia} are necessary. As a result, the overall time of one measuring cycle starting from time tₛₜₐ and ending on time t′ₙₑₓₜ is unnecessary long.

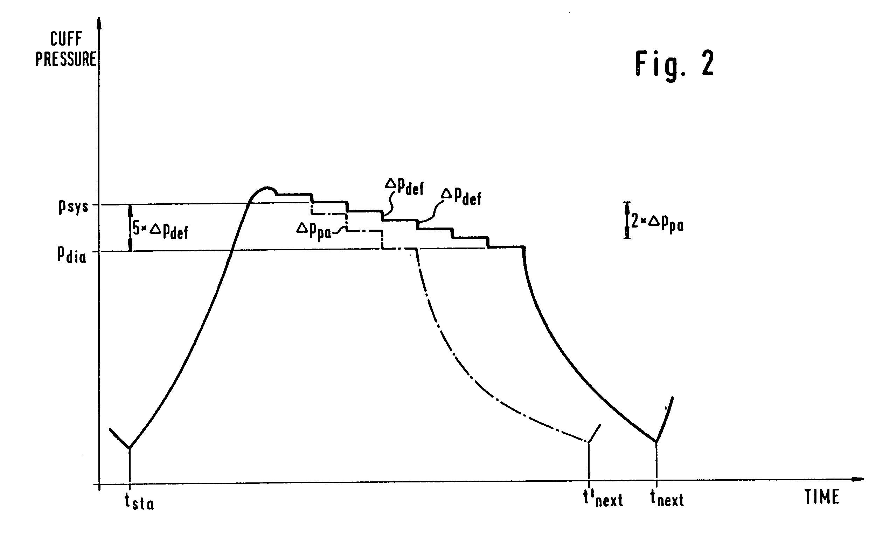

According to a preferred embodiment of the invention, using the formula (I) 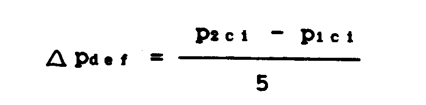
with
p_{2ci} = 180, representative to systolic pressure in the preceding cycle cᵢ, and
p_{1ci} = 100, representative to the diastolic pressure in cycle cᵢ,
five steps of size Δ p_{def} = 16 (= 16 mmHg = 2,128 kPa) occur. Further deflating steps are conducted above the systolic pressure and below the diastolic pressure. As a result, a shorter overall time for a measuring cycle starting at time tₛₜₐ and ending on time tₙₑₓₜ is achieved. The resulting eight pairs of peak amplitudes are sufficient to determine the envelope 10, out of which envelope 10 the systole, mean and diastole of the measuring cycle cᵢ₊₁ can be determined. Referring now to Figure 2, a cuff pressure/time graph is shown, demonstrating a blood pressure measuring cycle cᵢ₊₁ of an adult B, having hypotension with typical values of 70/50.

A dash-dotted line demonstrates the result of a conventional oscillometric method using predetermined incremental steps Δ pₚₐ = 1,064 kPa(8 mmHg). As a result, only two deflating steps occur between the systolic pressure p_{sys} and the diastolic pressure p_{dia}. An interpretation of the corresponding peak amplitudes or the envelopes thereof is nearly impossible and leads to pressure values with considerable errors.

According to the invention, using the equation (I) 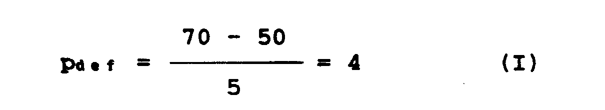
with
70 = p_{2ci} = p_{sys},
50 = p_{1ci} = p_{dia},
the adult B is measured with the same number of deflating steps (i.e. 5) as adult D, as illustrated within Figure 1.

As a result, the method according to the invention gives results of excellent and comparable relevancy.

The evaluation of subject's individual pressure values p_{2ci} and p_{1ci} can be varied. Also, the constant X can vary, depending on the accuracy of the equipment used. For example, an equipment with high sophisticated measuring means can use less deflating steps than more simple equipments.

However, according to the teaching of the present invention, all measurements done with one equipment leads to results being both adapted to the subject to be measured and being of same relevancy.

An apparatus according to the invention (not shown) comprise a cuff, inflating and deflating means, means for reducing cuff pressure and means for detecting of effects at the cuff as known in the art, for example as described within EP-A-0 208 520. The apparatus according to the invention further comprises processing means having storing means for storing pressure values p_{2ci} and p_{1ci} of subject within a cycle cᵢ. Said processing means determines the difference between p_{2ci} and p_{1ci} and subdivides said difference with factor x, thereby determining the size of incremental pressure reducing step p_{def} within cycle cᵢ₊₁. Said processing means provides said size values to the means for reducing cuff pressure, said means reduce cuff pressure within cycle c₁₊ᵢ with said size values Δ p_{def}.

Supervising means detect whether size value Δ p_{def} does not exceed upper and lower limits Δ p_{defmin} and Δ p_{defmax} .

## Claims

1. A method for measuring blood pressure, comprising the steps of
- applying a blood pressure cuff about a subject's limb containing an artery;
- inflating said cuff to a pressure pₘₐₓ above the systolic pressure p_{sys}, thereby occluding said artery;
- reducing cuff pressure from pₘₐₓ to at least a pressure pₘᵢₙ below the diastolic pressure p_{die}, thereby permitting an increasing flow through the progressively less occluded artery;
- conducting said reducing of cuff pressure with definite steps Δ p_{def};
- detecting of effects at the cuff caused by said increasing flow through said progressively less occluded artery;
- processing said detected effects in processing means and displaying said processed effects as subject's actual blood pressure values, and
- conducting a next subsequent blood pressure measuring cycle cᵢ₊₁ after a time interval,
characterized in that
the size of the incremental steps Δ p_{def} of a certain measuring cycle cᵢ₊₁ is a function of subject's actual blood pressure of the preceding measuring cycle cᵢ and which step Δ p_{def} is constant within said measuring cycle cᵢ₊₁.

2. A method according to claim 1, characterized in that the size of decremental step in cycle cᵢ₊₁ is 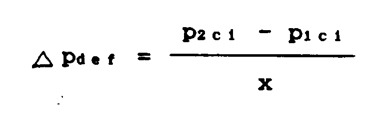 with
p_{2ci} ≦ pₘₐₓ in cycle cᵢ,
p_{1ci} ≧ pₘᵢₙ in cycle cᵢ,
p_{2ci} > p_{1ci} , and x = constant.

3. Method according to claim 2, characterized in that
p_{2ci} = p_{sysci}
p_{1ci} = p_{diaci}
3 ≦ x ≦ 6,
with
p_{sysci} = systolic pressure in cycle cᵢ,
p_{diaci} = diastolic pressure in cycle cᵢ.

4. A method according to any of the preceding claims, characterized in that the size of the decremental steps in a first cycle c₀ is adapted to pre-known subject's blood pressure data, being the basis of decremental steps in cycle c₀.

5. A method according to any of the preceding claims, characterized in that the size of incremental steps Δ p_{def} is
Δ p_{defmin} ≦ Δ p_{def} ≦ Δ p_{defmax}.

6. A method according to claim 5, characterized in that, in particular if measuring a human
Δ p_{defmin} is is about 0,5 kPa(4 mmHg), and
Δ p_{defmax} is about 2 kPa(16 mmHg).

7. An apparatus for automated blood pressure measuring, in particular for cyclic measuring, comprising
- an inflatable and deflatable pressure cuff, said cuff being applicable about a subject's limb containing an artery;
- means for inflating said cuff to a pressure pₘₐₓ above the systolic pressure p_{sys}, thereby occluding said artery;
- means for reducing cuff pressure from pₘₐₓ to at least a pressure pₘᵢₙ below the diastolic pressure p_{dia}, thereby permitting an increasing flow through the progressively less occluding artery, said means for reducing allow a stepwise reduction of cuff pressure, thereby stepwise deflating said cuff;
- means for detecting of effects at the cuff caused by said increasing flow through said progressively less occluded artery;
- processing means for processing said detected effects and for converting them to subject's actual blood pressure values, and
- means for initiating a subsequent next measuring cycle cᵢ₊₁,
characterized in that
the processing means further have storing means for storing pressure values of subject within cycle cᵢ, said processing means provide to said means for reducing cuff pressure the size of a decremental step Δ p_{def} in cycle cᵢ₊₁ as a function of said stored pressure values of cycle cᵢ and which step Δ p_{def} is kept constant within said measuring cycle cᵢ₊₁.

8. An apparatus according to claim 7, characterized in that said processing means store in cycle cᵢ at least two values p_{2ci}, p_{1ci}, and provide to said means for reducing cuff pressure in cycle cᵢ₊₁ decremental steps Δ p_{def} according to equation 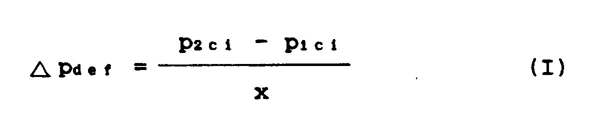 with
p_{2ci} ≦ pₘₐₓ in cycle cᵢ,
p_{1ci} ≧ pₘₐₓ in cycle cᵢ,
p_{2ci} > p_{1ci} , and x = constant.

9. Apparatus according to claim 8, characterized in that said processing means store in cycle cᵢ
p_{2ci} = p_{sysci},
p_{1ci} = p_{diaci},
with
p_{sysci} = systolic pressure in cycle cᵢ,
p_{diaci} = diastolic pressure within cycle cᵢ , and
3 ≦ x ≦ 6.

10. Apparatus according to claims 7, 8 or 9, characterized in that said processing means provide incremental steps p_{def} to said means for reducing cuff pressure within predetermined limits Δ p_{defmin} ≦ Δ p_{def} ≦ Δ p_{defmax}.

## Patentansprüche

1. Verfahren zur Blutdruckmessung, enthaltend die Schritte
- Anlegen einer Blutdruckmanschette um ein eine Arterie enthaltendes Gliedmaß eines Lebewesens;
- Aufpumpen der Manschette auf einen Druck pₘₐₓ oberhalb des systolischen Druckes p_{sys}, wodurch die Arterie verschlossen wird;
- Vermindern des Druckes der Manschette von pₘₐₓ auf zumindest einen Druck pₘᵢₙ unterhalb des diastolischen Druckes p_{dia}, wodurch ein zunehmendes Strömen durch die nach und nach weniger verschlossene Arterie möglich ist;
- Durchführen des Verminderns des Manschettendruckes mit definierten Stufen Δ p_{def};
- Erfassen der durch das zunehmende Strömen durch die nach und nach weniger verschlossene Arterie verursachten Effekte an der Manschette;
- Verarbeiten der erfaßten Effekte in Verarbeitungsmitteln und Anzeigen der verarbeiteten Effekte als aktuelle Blutdruckwerte des Lebewesens; und
- Durchführen eines nachfolgenden weiteren Blutdruckmeßzyklus cᵢ₊₁ nach einer Zeitspanne,
dadurch gekennzeichnet,
daß die Größe der Schrittstufen Δ p_{def} eines Meßzyklus cᵢ₊₁ eine Funktion des aktuellen Blutdruckes des Lebewesens im vorangegangenen Meßzyklus cᵢ ist, und daß die Stufe Δ p_{def} innerhalb des Meßzyklus cᵢ₊₁ konstant ist.

2. Verfahren nach Anspruch 1, dadurch gekennzeichnet, daß die Größe der Schrittstufen in Zyklus cᵢ₊₁ 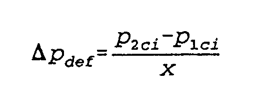 ist, wobei
p_{2ci} ≦ pₘₐₓ in Zyklus cᵢ,
p_{1ci} ≧ pₘᵢₙ in Zyklus cᵢ,
p_{2ci} > p_{1ci}, und x = konstant
ist.

3. Verfahren nach Anspruch 2, dadurch gekennzeichnet, daß
p_{2ci} = p_{sysci}
p_{1ci} = p_{diaci}
3 ≦ x ≦ 6,
ist, wobei
p_{sysci} = systolischer Druck in Zyklus cᵢ
p_{diaci} = diastolischer Druck in Zyklus c₁
ist.

4. Verfahren nach einem der vorhergehenden Ansprüche, dadurch gekennzeichnet, daß die Größe der Schrittstufen in einem. ersten Zyklus c₀ bekannten Blutdruckdaten des Lebewesens angepaßt ist, die wiederum Basis der Schrittstufen in Zyklus c₀ sind.

5. Verfahren nach einem der vorhergehenden Ansprüche, dadurch gekennzeichnet, daß die Größe der Schrittstufen Δ p_{def}
Δ p_{defmin} ≦ Δ p_{def} ≦ Δ p_{defmax}
ist.

6. Verfahren nach Anspruch 5, dadurch gekennzeichnet, daß, insbesondere bei Messungen am Menschen,
△ P_{defmin} etwa 0,5 kPa (4 mmHg) und
△ P_{defmax} etwa 2 kPa (16 mmHg)
ist.

7. Vorrichtung zur automatischen Blutdruckmessung, insbesondere für zyklische Messungen, mit
- einer aufpumpbaren und ablaßbaren Druckmanschette, wobei die Manschette um ein eine Arterie enthaltendes Gliedmaß eines Lebewesens legbar ist;
- Mittel zum Aufpumpen der Manschette auf einen Druck pₘₐₓ oberhalb des systolischen Druckes p_{sys}, wobei die Arterie verschlossen wird;
- Mittel zum Vermindern des Druckes der Manschette von pₘₐₓ auf zumindest einen Druck pₘᵢₙ unterhalb des diastolischen Druckes p_{dia}, wodurch ein zunehmendes Strömen durch die nach und nach weniger verschlossene Arterie möglich ist, wobei die Mittel ein stufenweises Vermindern des Druckes der Manschette ermöglichen, so daß die Manschette stufenweise abgelassen wird;
- Mittel zum Erfassen der durch das zunehmende Strömen durch die nach und nach weniger verschlossene Arterie verursachten Effekte an der Manschette;
- Verarbeitungsmittel zum Verarbeiten der erfaßten Effekte und zum Umwandeln dieser in aktuelle Blutdruckmeßwerte des Lebewesens, und
- Mittel zum Veranlassen eines nachfolgenden weiteren Meßzyklus cᵢ₊₁,
dadurch gekennzeichnet,
daß die Verarbeitungsmittel Speichermittel zum Speichern von Druckwerten des Lebewesens in Zyklus cᵢ aufweisen, daß die Verarbeitungsmittel den Mitteln zum Vermindern des Manschettendruckes die Größe der Schrittstufen △ p_{def} in Zyklus cᵢ₊₁ als eine Funktion der gespeicherten Druckwerte von Zyklus cᵢ geben, und daß die Stufe △ p_{def} im Meßzyklus cᵢ₊₁ konstant gehalten ist.

8. Vorrichtung nach Anspruch 7, dadurch gekennzeichnet, daß die Verarbeitungsmittel in Zyklus cᵢ zumindest zwei Werte p_{2ci}, p_{1ci} speichern, und den Mitteln zum Vermindern des Manschettendruckes in Zyklus cᵢ₊₁ Schrittstufen △ p_{def} entsprechend der Gleichung 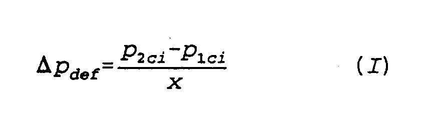 geben, wobei
p_{2ci} ≦ pₘₐₓ in Zyklus cᵢ,
P_{1ci} ≧ pₘₐₓ in Zyklus cᵢ,
p_{2ci} > p_{1ci}, und x = konstant
ist.

9. Vorrichtung nach Anspruch 8, dadurch gekennzeichnet, daß die Verarbeitungsmittel in Zyklus cᵢ
P_{2ci} = P_{sysci},
p_{1ci} = p_{diaci} speichern,
wobei
p_{sysci} = systolischer Druck in Zyklus cᵢ,
p_{diaci} = diastolischer Druck in Zyklus cᵢ, und
3 ≦ x ≦ 6
ist.

10. Vorrichtung nach den Ansprüchen 7, 8 oder 9, dadurch gekennzeichnet, daß die Verarbeitungsmittel den Mitteln zum Vermindern des Manschettendruckes Schrittstufen △ p_{def} in vorbestimmten Grenzen △ p_{defmin} ≦ △ p_{def} ≦ △ p_{defmax} geben.

## Revendications

1. Procédé pour la mesure de la pression sanguine, comportant les étapes de
- application d'une manchette de pression sanguine autour d'un membre du patient, contenant une artère;
- gonflage de ladite manchette jusqu'à une pression pₘₐₓ supérieure à la pression systolique p_{sys}, bouchant ainsi ladite artère;
- réduction de la pression de la manchette depuis pₘₐₓ jusqu'à, au moins, une pression pₘᵢₙ inférieure à la pression diastolique p_{dia}, autorisant ainsi un débit croissant à travers l'artère progressivement de moins en moins bouchée;
- conduite de ladite réduction de pression de la manchette en pas définis △ p_{def};
- détection des effets sur la manchette dus au débit croissant à travers l'artère progressivement de moins en moins bouchée;
- traitement desdits effets détectés par un moyen de traitement et affichage desdits effets traitées comme yaleurs de la pression sanguine réelle du patient, et
- commande d'un cycle de mesure suivant cᵢ₊₁ de la pression sanguine, après un certain intervalle de temps.
caractérisé en ce que
la taille des pas décrémentaux △ p_{def} d'un certain cycle de mesure Cᵢ₊₁ est une fonction de la pression sanguine réelle du patient au cours d'un cycle de mesure précédent Cᵢ, et ce pas △ p_{def} est constant à l'intérieur dudit cycle de mesure cᵢ₊₁.

2. Procédé selon la revendication 1, caractérisé en ce que la taille des pas décrémentaux pendant le cycle cᵢ est 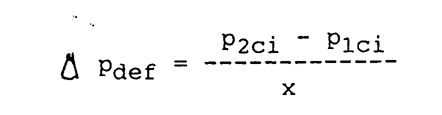 avec
p_{2ci} ≦ pₘₐₓ pendant le cycle cᵢ
p_{1ci} ≧ pₘᵢₙ pendant le cycle cᵢ,
p_{2ci} > p_{1ci} et x = constante

3. Procédé selon la revendication 2, caractérisé en ce que
p_{2ci} = p_{sysci},
p_{1ci} = p_{disci} et
3 ≦ x ≦ 6.
avec
p_{sysci} = pression systolique pendant le cycle cᵢ,
p_{disci} = pression diastolique pendant le cycle cᵢ.

4. Procédé selon l'une quelconque des revendications précédentes caractérisé en ce que la taille des pas décrémentaux dans un premier cycle c₀ est adaptée aux données de pression sanguine du patient déjà connues, qui constituent la base des pas décrémentaux dans le cycle c₀

5. Procédé selon l'une quelconque des revendications précédentes, caractérisé en ce que la taille des pas décrémentaux △ p_{def} est
△ p_{defmin} ≦ △ p_{def} ≦ △ p_{defmax}

6. Procédé selon la revendication 5, caractérisé en ce que, en particulier, si l'on mesure un humain
△ p_{defmin} est d'environ 0,5 kPa (4 mmHg), et
△ p_{defmax} est d'environ 2 kPa (16 mmHg).

7. Appareil pour la mesure automatique de la pression sanguine, en particulier pour la mesure cyclique, comportant
- une manchette à pression gonflable et dégonflable, ladite manchette pouvant s'appliquer autour d'un membre du patient, contenant une artère;
- un moyen de gonflage de ladite manchette jusqu'à une pression pₘₐₓ supérieure à la pression systolique p_{sys}, bouchant ainsi ladite artère;
- des moyens de réduction de la pression de la manchette depuis pₘₐₓ jusqu'à, au moins, une pression pₘᵢₙ inférieure à la pression diastolique p_{dia}, autorisant ainsi un débit croissant à travers l'artère progressivement de moins en moins bouchée, lesdits moyens de réduction permettent une réduction par pas de la pression de la manchette, dégonflant ainsi ladite manchette par pas;
- un moyen de détection des effets sur la manchette, provoqués par ledit débit croissant à travers l'artère progressivement de moins en moins bouchée;
- un moyen de traitement destiné à traiter lesdits effets détectés et à convertir lesdits effets en valeurs de la pression sanguine réelle du patient, et
- un moyen destiné à commander le cycle de mesure suivant cᵢ₊₁.
caractérisé en ce que
le moyen de traitement comporte également un moyen de stockage destiné à stocker les valeurs de pression du patient à l'intérieur d'un cycle cᵢ, ledit moyen de traitement fournit audit moyen de réduction de la pression de la manchette la taille d'un pas décrémental △ p_{def} à l'intérieur du cycle cᵢ₊₁ comme fonction desdites valeurs de pression stockées du cycle cᵢ et le pas △ p_{def} est maintenu constant à l'intérieur dudit cycle de mesure cᵢ₊₁.

8. Appareil selon la revendication 7, caractérisé en ce que ledit moyen de traitement stocke dans le cycle cᵢ, au moins deux valeurs p_{2ci}, p_{1ci}, et fournit audit moyen de réduction de la pression de la manchette dans le cycle cᵢ₊₁, des pas décrémentaux △ p_{def} selon l'équation 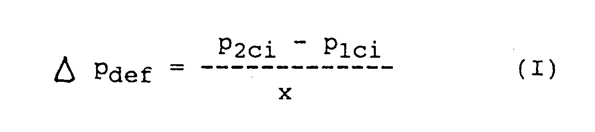 avec
p_{2ci} ≦ pₘₐₓ pendant le cycle cᵢ
p_{1ci} ≧ pₘᵢₙ pendant le cycle cᵢ,
p_{2ci} > p_{1ci} et x = constante

9. Appareil selon la revendication 8, caractérisé en ce que ledit moyen de traitement stocke pendant le cycle cᵢ
p_{2ci} = p_{sysci},
p_{1ci} = p_{disci},
avec
p_{sysci} = pression systolique pendant le cycle cᵢ,
p_{disci} = pression diastolique pendant le cycle cᵢ, et
3 ≦ x ≦ 6.

10. Appareil selon les revendications 7, 8 ou 9, caractérisé en ce que ledit moyen de traitement fournit des pas incrémentaux △ p_{def} audit moyen de réduction de la pression de la manchette dans des limites prédéterminées △ p_{defmin} ≦ △ p_{def} ≦ △ p_{defmax}.
